# EUROPEAN PATENT SPECIFICATION

(11) **EP 4 313 205 B1**
(45) Date of publication and mention of the grant of the patent: **04.06.2025**
(21) Application number: 22720927.7
(22) Date of filing: 25.03.2022
(51) Int. Cl.: A61M 1/36

(54) **DEVICES FOR FISTULA-FREE HEMODIALYSIS**
VORRICHTUNGEN FÜR FISTELLOSE HEMODIALYSE
DISPOSITIFS POUR L'HÉMODIALYSE LIBRE DE FISTULE

(30) Priority: 30.03.2021 US 202163167771 P; 31.03.2021 US 202163168276 P; 31.03.2021 US 202163168277 P; 15.07.2021 US 202163222062 P
(43) Date of publication of application: 07.02.2024
(60) Divisional application: 23195489.2
(73) Proprietor: GRUNWALD, Sorin, 93500 Pantin (FR)
(72) Inventor: GRUNWALD, Sorin, 93500 Pantin (FR)
(86) International application number: PCT/EP2022/057987
(87) International publication number: WO 2022/207505

(56) References cited:
- WO-A1-2020/004672
- US-A1- 2005 143 758
- US-A1- 2014 100 510
- US-A1- 2020 114 060
- US-B2- 8 845 573

## Description

### REFERENCES CITED

### CROSS REFERENCE TO RELATED APPLICATIONS

This application is a division of PCT Application No. PCT/EP2022/057987 filed on 25 March 2022, International Publication Number WO 2022/207505 A2 and claims priorities to:
1. U.S. Provisional Patent Application Serial No. US 63/167,771 filed on March 30, 2021 (Needleless transcutaneous port)
2. U.S. Provisional Patent Application Serial No. US63/168,276, filed on March 31, 2021 (Anastomosis device and method for percutaneous delivery thereof)
3. U.S. Provisional Patent Application Serial No. US63/168,277, filed on March 31, 2021 (Devices and methods for Hemodialysis)
4. U.S. Provisional Patent Application Serial No. US 63/222,062, filed on July 15, 2021 (Devices and methods for sutureless anastomosis).

### U.S. PATENT DOCUMENTS

| | | | |
|---|---|---|---|
| 3,628,813 | 12/1971 | Lee | Fluid connecting device |
| 4,007,743 | 2/1977 | Blake | Opening mechanism for umbrella-like intravascular shunt defect closure device |
| 4,352,358 | 10/1982 | Angelchik | Apparatus for effecting anastomotic procedures |
| 4,353,368 | 10/1982 | Slovak et al. | Device for hemodialysis |
| 4,569,675 | 2/1986 | Prosl et al. | Transcutaneous infusion system |
| 4,892,518 | 1/1990 | Cupp et al. | Hemodialysis |
| 4,968,422 | 11/1990 | Runge et al. | Pulsatile flow hemodialysis |
| 5,035,702 | 7/1991 | Taheri | Method and apparatus for providing an anastomosis |
| 5,342,393 | 8/1994 | Stack | Method and device for vascular repair |
| 5,456,712 | 10/1995 | Maginot | Graft and stent assembly |
| 5,562,617 | 10/1996 | Finch, Jr. et al. | Implantable vascular device |
| 5,755,775 | 5/1998 | Trerotola et al. | Percutaneous stent-graft and method for delivery thereof |
| 5,755,778 | 5/1998 | Kleshinski | Anastomosis device |
| 5,861,003 | 1/1999 | Latson et al. | Apparatus and method for occluding a defect or aperture within body surface |
| 5,911,706 | 6/1999 | Estabrook et al. | Device for subcutaneous accessibility |
| 5,944,688 | 8/1999 | Lois | Implantable hemodialysis access port assembly |
| 6,036,702 | 3/2000 | Bachinski et al | Medical grafting connectors and fasteners |
| 6,231,541 | 5/2001 | Kawamura | No-needle blood access device for hemodialysis and no-needle connecting cannula assembly |
| 6,312,446 | 11/2001 | Huebsch et al. | Apparatus and method for closing a septal defect |
| 6,402,764 | 6/2002 | Henricksen et al. | Everter and threadthrough system for attaching graft vessel to anastomosis device |
| 6,428,550 | 8/2002 | Vargas et al. | Sutureless closure and deployment system for connecting blood vessels |
| 6,648,901 | 11/2003 | Fleischman et al. | Anastomosis system |
| 6,719,781 | 4/2004 | Kim | Catheter apparatus having an improved shape-memory alloy cuff and inflatable on-demand balloon for creating a bypass graft in-vivo |
| 6,776,785 | 8/2004 | Yencho et al. | Implantable superelastic anastomosis device |
| 7,585,306 | 9/2009 | Abbott et al. | Anastomosis device, tools and methods of using |
| 7,762,977 | 7/2010 | Porter et al. | Device and methods for vascular access |
| 8,277,465 | 10/2012 | Beane et al. | Apparatus and method for connecting a conduit to a hollow vessel |
| 8,747,344 | 6/2014 | Khan | Hybrid arteriovenous shunt |
| 8,845,573 B2 | 9/2014 | Häusler et al. | Implantable access for removal and/or return of fluids |
| 8,900,288 | 12/2014 | Chobotov et al. | Advanced endovascular graft delivery system and method of treatment |
| 8,926,591 | 1/2015 | Schutz et al. | Implantable vascular access |
| 9,247,930 | 2/2016 | Coleman et al. | Device and methods for occluding or promoting fluid flow |
| 9,597,443 | 3/2017 | Yevzlin et al. | Anastomotic connector |
| 9,820,743 | 11/2017 | Asfora et al. | Sutureless vascular anastomosis connection |
| 10,702,688 | 7/2020 | Mason et al. | Vascular access device |
| 10,166,321 | 1/2019 | Casiello et al. | High-flow port and infusion needle systems |
| 10,751,056 | 8/2020 | Porter | Methods and apparatus for percutaneous bypass graft |
| 10,773,010 | 9/2020 | Phillips et al. | Subcutaneous vascular access ports and related systems and methods (needle guiding devices) |
| 10,835,366 | 11/2020 | Donadio, III et al. | Arterial and venous anchor devices forming an anastomotic connector and system for delivery |
| 10,835,663 | 11/2020 | Peh et al. | Subcutaneous implantable device for guiding a vascular access member and method for vascular access |

### FOREIGN PATENT DOCUMENTS

| | | | | |
|---|---|---|---|---|
| WO | 97/42878 | 11/1997 | Kotula et al. | Percutaneous catheter directed intravascular occlusion devices |
| WO | 2020/004672 A1 (KK ADVANCE [JP]) 2 January 2020 (2020-01-02) | | | reference cited in the written opinion of the International Searching Authority [19] |

### OTHER PUBLICATIONS

1. Agarwal A.K. et al., Innovations in vascular access for hemodialysis, Kidney International (2019) 95, 1053-1063
2. Allon M., Vascular Access for Hemodialysis Patients - New Data Should Guide Decision Making, American Society of Nephrology, CJASN Vol 14 June, 2019
3. Canaud B. et al., Vascular Access Management for Haemodialysis: A Value-Based Approach from NephroCare Experience, IntechOpen 2019
4. Ilie V. et al., Sutureless Microvascular Anastomosis: Literature Review, International Microsurgery Journal, 2019; 3(2):1
5. Mallios A. et al., Early results of percutaneous arteriovenous fistula creation with the Ellipsys Vascular Access System, Journal of Vascular Surgery, 2018
6. Al-Jaishi A.A. et al., Complications of the Arteriovenous Fistula: A Systematic Review, J Am Soc Nephrol 28: 1839-1850, 2017
7. Al-Balas A. et al., The Clinical and Economic Effect of Vascular Access Selection in Patients Initiating Hemodialysis with a Catheter, J Am Soc Nephrol 28: 3679-3687, 2017
8. Al Shakarchi J. et al., Early cannulation grafts for hemodialysis: a systematic review, J Vasc Access 2015; 16(6): 493-497
9. Parisotto, M.T., et al., Cannulation technique influences arteriovenous fistula and graft survival, Kidney International (2014) 86, 790-797
10. Ebner A. et al., Minimally Invasive Sutureless Anastomosis of an AV Hemodialysis Graft, Endovascular Today, November 2014
11. Stolic R, Most Important Chronic Complications of Arteriovenous Fistulas for Hemodialysis, Medical Principles and Practice 2013; 22:220-228
12. Vachharajani T.J., Atlas of Dialysis Vascular Access, Wake Forest University, School of Medicine, 2010
13. Hadaway L. et al., Needleless Connectors: A Primer on Terminology, Journal of Infusion Nursing, January 2010
14. Misra M., The basics of hemodialysis equipment, Hemodialysis International 2005; 9:3--36
15. Twardowski Z.J. et al., Blood Flow, Negative Pressure, and Hemolysis During Hemodialysis, Home Hemodial Int, Vol 3, 45-50. 1999
16. Foran R.F., Quinton-Scribner Cannulas for Hemodialysis - Review of Four Years' Experience, The Western Journal of Medicine, March 1970, 112-3
17. Brescia M.J. et al., Chronic hemodialysis using venipuncture and a surgically created arteriovenous fistula, N Engl J Med. 1966; 275:1089-1092
18. Clark P.B., Routine Use of Scribner Shunt for Haemodialysis, British Medical Journal, May 1966: 1200 - 1202

### TECHNICAL FIELD

The present invention relates generally to devices and methods that can be used for vascular access for hemodialysis. More specifically, the present invention relates to devices and methods that can be delivered percutaneously and suturelessly and that do not require an arteriovenous fistula, an arteriovenous graft, or a central venous catheter for vascular access (fistula-free hemodialysis). Further, the present invention relates to devices for improved and needleless connection of a patient to a dialyzer.

### BACKGROUND

### Clinical Background

Worldwide, hemodialysis (HD) remains the prevalent dialysis modality for more than 2 million patients with end-stage renal disease. The health care expenditure to treat end-stage renal disease has increased to approximately $34 billion in 2015 in the United States alone. A significant portion of this expense is related to the establishment and maintenance of vascular access (VA) for HD [1].

The 2006 Kidney Disease Outcomes Quality Initiative (KDOQI) VA guidelines state that "options for fistula placement should be considered first." However, more recent publications have reported 30%-60% arteriovenous fistula (AVF) nonmaturation rates for AVFs that cannot be used or require assisted maturation [2]. Although the AVF is currently considered the ideal model of VA for HD, AVFs are the source of complications, the most important being lymphedema, infection, aneurysm, stenosis, congestive heart failure, steal syndrome, hand ischemia and thrombosis. Fistula complications are associated with morbidity, mortality, and a high economic burden [6]. Several studies indicate that about 30% of HD hospitalizations are caused by VA construction and complications of vascular access [11]. Endovascular creation has simplified the creation of AVFs using either radiofrequency (everlinQ, TVA Medical, Austin, TX, USA) or thermal resistance (Ellipsys, Avenu Medical, San Juan Capistrano, CA, USA) arteriovenous anastomosis devices [1], [5]. However, many of clinical issues and complications related to AVFs, as mentioned above, have remained unaddressed.

If a patient is not a suitable candidate for AVF, arteriovenous grafts (AVG) is the second vascular access option for HD. Compared to the AVF, the AVG has better mechanical strength, earlier use, and primary failure rates but also increased risk of developing graft stenosis, a fivefold increase in infection risk, a poorer long-term patency, higher levels of complications and requires more interventions than AVF [2]. Central venous catheters (CVC) are also used in specific cases for starting hemodialysis because they provide immediate vascular access. However, prolonged CVC use results in high risk of CVC-related bloodstream infections. CVCs are also associated with a higher risk of central vein stenosis and decreased survival rates [2].

### Related Art

The creation of an AVF for long-term HD in 1966 by Cimino and Brescia [17] was the first major innovation after the Scribner shunt first described in 1961 and was followed by the development of arteriovenous grafts and hemodialysis catheters. To date, the innovations in vascular access for hemodialysis have focused on arteriovenous fistulas, arteriovenous grafts, central venous catheters and the process of healthcare. Innovations related to arteriovenous fistulas have focused on: a) endovascular creation; b) (sutureless) anastomotic devices; c) maturation facilitators; and d) superificialization. Innovations related to arteriovenous grafts have focused on: a) early access AVG; b) heparin-bonded AVG; c) hybrid catheter graft; d) hybrid graft-stent; and e) tissue bioengineered vessels. Innovations related to central venous catheters have focused on: a) catheter tip design; b) catheter coating; and c) catheter lock solutions. Innovations related to the process of healthcare have focused on: a) patient-centric care; b) value versus volume-based care; c) preemptive versus reactive care; and d) evidence-based care to reduce health care-associated infections [1].

Foran [16] and Clark [18] describe the use of a device, the Scribner shunt, that provides separate arterial and venous access for HD. Some of the most important benefits of the Scribner shunt are: a) the ease-of-use for repetitive HD access compared to new cannulations; and b) the ability to use the shunt for HD access immediately after implantation. Some of the limitations of the Scribner shunt were: a) the fact that the device was an extracorporeal device which lead to a higher risk of infection, in particular at the points of insertion; b) the arterial and venous indwelling segments of the devices caused thrombosis and stenosis; c) the high complexity of the surgical insertion and fixation of the cannula tip, particularly in the artery; and d) the material of the device caused clotting and progressive deterioration of the cannula function.

In U.S. Pat. No. 6,231,541 Kawamura describes a no-needle blood access device for HD comprising an artificial conduit whose opposite ends are anastomosed to a target artery or vein. The device contains a transcutaneous component that allow for connecting the subcutaneous conduit to an extracorporeal HD machine. Some of the limitations of Kawamura's disclosure are: the disclosed device requires a fistula or graft to connect a vein to an artery, the fistula/graft is created through open surgery and sutured to the target vessels; there is only one skin access point for both the arterial and venous lines for hemodialysis limiting the choice of target blood vessels and access points; the use of disclosed shutters to open and close conduits leads to blood loss when connecting a dialyzer; the long cannulas inserted in the blood stream increase the risk of infections; and once implanted, the device cannot be replaced if it fails in time.

In U.S. Pat. No. 6,719,781 Kim describes a catheter apparatus, an improved introducer system, and a methodology for creating a bypass using a prepared shape-memory alloy cuff and a graft segment in tandem as a shunt. Some of the limitations of Kim's disclosure are: the cuff is fixed to the graft by the surgeon through suturing during the procedure and the fixing means can cause tissue damage; the cuff expands based on thermal properties and needs cooling until deployment; the disclosed device can only be used for large arteries (aorta) at one end of the graft while the other end of the graft is connected surgically with sutures by the surgeon in a standard surgical procedure; and, in general, grafts cannot be connected to a dialyzer considering the conduit sizes and fluid pressures required by the hemodialysis process.

In U.S. Pat. No. 6,776,785 Yencho et al. describe a one-piece anastomosis device formed of a superelastic or pseudoelastic material which self-deforms or self-deploys from an insertion configuration to a tissue holding configuration. The self-deploying anastomosis device does not rely on a temperature transformation to achieve deployment. Some of the limitations of Yencho's disclosure are: the disclosed device can only be used to connect a tissue graft and cannot be used to connect a catheter or other semi-rigid conduit and, thus, cannot be used for hemodialysis; the risk of damaging the blood vessel is high during the deployment procedure due to the lack of maneuverability, and the device cannot be placed without direct visualization of the target vessel.

In U.S. Pat. No. 7,585,306 Abbott et al. describe anastomosis devices, tools and methods of performing sutureless anastomosis. Anastomosis devices are provided for fixing a first conduit to a second conduit in an anastomosis, where the conduits are joined by interfacing their inner walls together. Some of the limitations of Abbott's disclosure are: the devices can be used only for large blood vessels, e.g. aorta, because of the reverted graft attaching mechanism; the device cannot be connected to a catheter or other semi-rigid conduit and, thus, cannot be used for hemodialysis;
In U.S. Pat. No. 8,845,573 Häusler et al. disclose an implantable access device for removal and/or return of fluids to a patient, comprising a casing attached to an internal conduit to be connected to a lumen of the patient. The disclosed device comprises a fixation unit arranged for fixation of the device to a bone of the patient. However, the disclosure does not mention how the device can be implanted subcutaneously without the attachment to a patient bone. Further, the disclosure does not mention a variable inner lumen profile for blood flow control, nor does it mention how it can be used for hemodialysis transcutaneous access.

In U.S. Pat. No. 9,247,930 Coleman et al. describe devices and sutureless percutaneous methods for occluding or promoting fluid flow through openings. In an exemplary embodiment an occlusion device is provided having an outer elongated tubular body that is configured to expand and form proximal and distal wings proximate to opposed ends of the opening. The device can include a component to occlude flow through the tubular body and thus through the opening. Some of the limitations of Coleman's disclosure are: the disclosed device cannot be used to promote flow in small vessels because of the inherent wall thickness of the disclosed device structure. A device with a small outer diameter and an even smaller inner diameter cannot ensure the fluid debits required for hemodialysis, i.e., 300 - 600 ml/min; in addition, when promoting flow, certain disclosed device components must reside inside the target blood vessel obstructing flow in said blood vessel. Thus, the device can only be used for bypass procedure where the blood flow is already obstructed in the target vessel. Moreover, the disclosed device is made of a solid stainless steel structure that is too heavy to attach to small vein walls that would collapse under the device weight.

In U.S. Pat. No. 9,597,443 Yevzlin et al. describe an anastomotic connector comprising a tubular access port and an anchor with a plurality of fingers to be extended in a blood vessel connected to the tubular access port. Some of the limitations of Yevzlin's disclosure are: the disclosure does not allow for percutaneous deployment of a catheter pre-connected to the disclosed anastomotic connector thus limiting the choice of anastomosis locations to those directly accessible by open surgery; the proximal end of the anastomotic connector that resides outside the blood vessel is fixed in shape and perpendicular to the longitudinal axis of the blood vessel, said characteristics further limiting the maneuverability of the device and the ability to connect it to smaller blood vessels.

### Clinical Need

What is needed are improved devices and methods for vascular access for hemodialysis that reduce the risks associated with establishing and maintaining vascular access, decrease time to first use, increase long-term patency, minimize complications, simplify the hemodialysis procedure, and reduce the burden on patients and on the healthcare system.

### SUMMARY

Devices and methods for fistula-free-vascular access for hemodialysis are disclosed herein. In exemplary embodiments, devices are provided including a transcutaneous access port for needlelessly connecting a patient to a dialyzer, an intracorporeal conduit providing a fluid path between said transcutaneous port and a blood vessel, and a device anchor, said device anchor permitting the sutureless anastomosis of said intracorporeal conduit to said blood vessel. In exemplary embodiments, methods are provided for creating fistula-free vascular access for hemodialysis and performing hemodialysis procedures without needle punctures.

In one exemplary embodiment, an implantable transcutaneous access port disclosed herein comprises a port body with an inner lumen and a subcutaneous segment attachable to an intracorporeal conduit, wherein the longitudinal axis of said subcutaneous segment and the longitudinal axis of said port body are at a variable angle with respect to each other in order to adapt to the anatomy of the access and implant locations and wherein said inner lumen has different profiles such as to provide variable blood flow characteristics adapted to either arterial or venous blood flow and, wherein the port body can be connected to an extracorporeal connector and to a dialyzer.

In one exemplary embodiment, an intracorporeal conduit disclosed herein comprises a distal more rigid segment and a proximal less rigid segment, said proximal less rigid segment being attached to a transcutaneous access port and said distal more rigid segment being fixedly attached to and within a tubular device anchor, wherein said distal more rigid segment extends through the inner lumen of said device anchor such that the distal end of said segment and the distal end of said device anchor are flush, and wherein the outer diameter of said distal more rigid segment of said conduit is equal to or slightly smaller than the inner diameter of said device anchor.

In one exemplary embodiment, a fistula-free hemodialysis method disclosed herein consists of percutaneously deploying a first said intracorporeal conduit and anastomosing it to an artery for creating arterial access and a second said intracorporeal conduit and anastomosing it to a vein for creating venous access and independently connecting each of said devices respectively to the arterial and venous lines of said dialyzer using said transcutaneous ports.

The contributions of the present invention in addressing current hemodialysis needs with respect to prior art are manyfold. The devices disclosed in the present invention can be delivered percutaneously and without employing sutures and provide independent access to arteries and veins. The methods disclosed herein allow for the creation of fistula-free vascular access for hemodialysis without the need for an arteriovenous fistula, arteriovenous graft or central venous catheter. Thus, the risks and issues associated with the creation and maintenance of vascular access for hemodialysis are significantly reduced. Moreover, the devices disclosed herein provide access for required hemodialysis debits of 300-600 ml/min to and from blood vessels as small as 2-3 mm in diameter, thus increasing the number of choices for vascular access points in a patient's vasculature. Further, the devices and methods disclosed herein allow for a needleless connection between a patient and a dialyzer, thus simplifying and decreasing the burden of the hemodialysis procedure in hospitals and homecare settings.

### BRIEF DESCRIPTION OF THE DRAWINGS

FIG. **1** illustrates an example embodiment of a method of use of the present invention.
FIG. **2** illustrates an example embodiment of a transcutaneous access port according to the present invention.
FIG. **23** illustrates an example embodiment of a device and method for needlelessly connecting a patient to a dialyzer or to another external device according to the present invention.
FIG. **24** illustrates another example embodiment of a transcutaneous access port placed subcutaneously according to the present invention.
FIG. **25** illustrates another example embodiment of a transcutaneous access port placed subcutaneously and comprising an adaptor for several predefined needle access points (buttonholes) according to the present invention.

### DETAILED DESCRIPTION

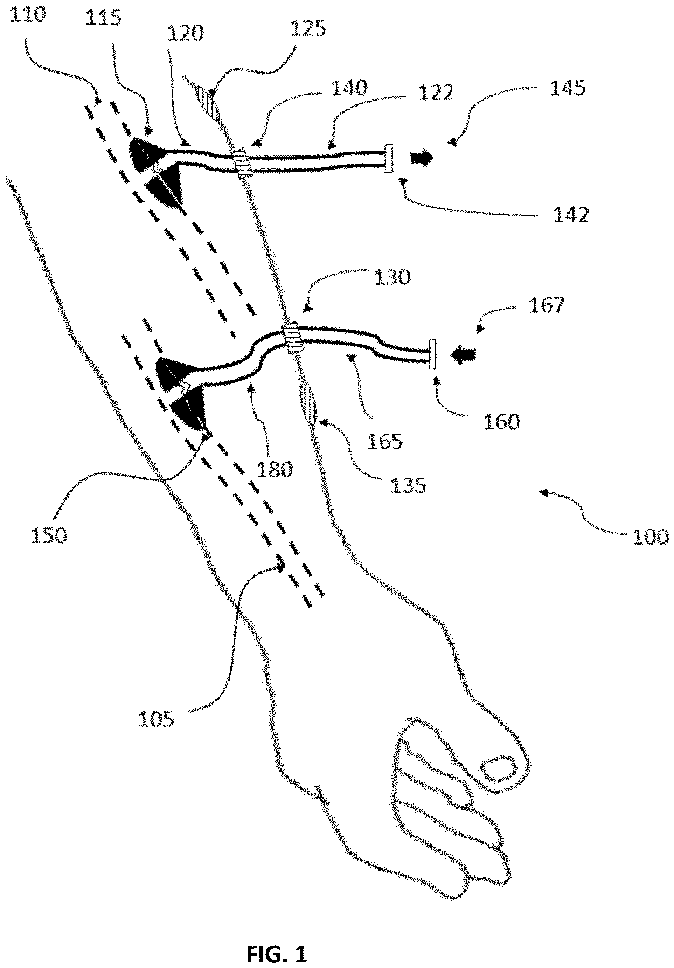

FIG. **1** illustrates an exemplary context of use **100** of the present invention. In order to create vascular access for hemodialysis, a first intracorporeal conduit **120** is deployed percutaneously through a skin area **125** and attached to the wall of a conduit in the body, e.g., to the wall of a blood vessel or graft **110** using the device anchor **115** as described further herein. In one embodiment of the present invention, blood vessel **110** is an artery. In one embodiment of the present invention, the conduit **120** is attached to transcutaneous access port **140** in order to facilitate the connection of conduit **120** to an external device using conduit **122** and connector **142.** In one embodiment of the present invention, transcutaneous port **140** is attached to the patient's skin. In one embodiment of the present invention, conduit **122** can be attached to and detached from transcutaneous access port **140.** In one embodiment of the present invention, connector **142** is attached to the arterial line of a hemodialysis machine (dialyzer) and blood can be drawn **(145)** from artery **110** into the dialyzer during the hemodialysis procedure.

A second intracorporeal conduit **180** is deployed percutaneously through a skin area **135** and attached to the wall of a conduit in the body, e.g., to the wall of a blood vessel or graft **105** using the device anchor **150** as described further herein. In one embodiment of the present invention blood vessel **105** is a vein. In one embodiment of the present invention, conduit **180** is attached to transcutaneous port **130** in order to facilitate the connection of conduit **180** to an external device using conduit **165** and connector **160.** In one embodiment of the present invention, transcutaneous access port **130** is attached to the patient's skin. In one embodiment of the present invention, conduit **165** can be attached to and detached from transcutaneous access port **130.** In one embodiment of the present invention, connector **160** is attached to the venous line of a hemodialysis machine (dialyzer) and blood can be infused (returned) **(167)** into vein **105** from the dialyzer during the hemodialysis procedure.

In another embodiment of the present invention, a first and a second intracorporeal conduits can be attached (anastomosed) at different locations on the same conduit in the body, e.g., on the same vein or a graft.

In another embodiment of the present invention, only one intracorporeal conduit and transcutaneous port is deployed, e.g., to a vein or graft for single needle hemodialysis.

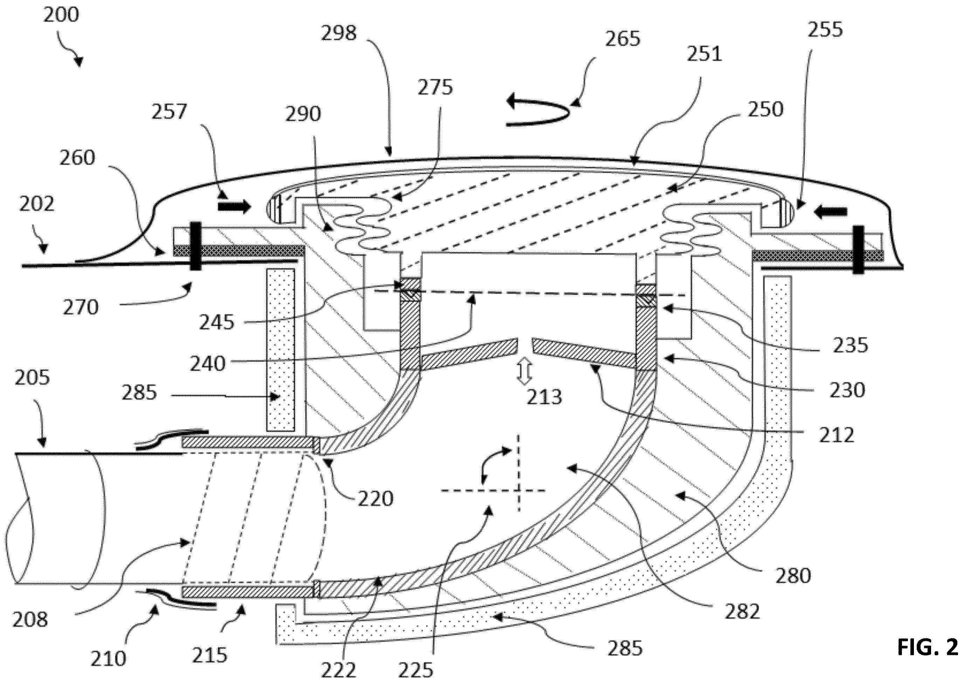

FIG. **2** illustrates an example embodiment of a transcutaneous access port **200** according to the present invention. Said port allows for repetitive needles access to an intracorporeal conduit **205** in a patient's body through the patient's skin **202.** A subcutaneous segment **215** of port **200** can be connected to the proximal end of said conduit **205** as described herein. The distal end of conduit **205** can be indwelling in a patient's blood vessel or anastomosed to another conduit of the human body, e.g., to a blood vessel or to an arteriovenous graft.

According to one embodiment of the present invention, the port body **280** is connected to the implanted intracorporeal conduit **205** by pushing the conduit **205** into the segment **215** of the port body. In another embodiment of the present invention, the conduit **205** can be pushed over the segment **215** of the port body. In another embodiment of the present invention, the conduit **205** and the inner wall of the segment **215** are made with matching threads **208,** such that conduit **205** can be threaded into the segment **215.** In one embodiment of the present invention, the conduit **205** is pushed or threaded into the segment **215** up to a stop **220.** The width of stop **220** matches the wall size of conduit **205** such that the inner lumen presents no obstacle to the flow through the conduit and laminar flow is ensured. After inserting the conduit **205** into or over segment **215** of the port body, a band or ring **210** is deployed to additionally secure in place the connection between the conduit **205** and the port body segment **215.**

In one embodiment of the present invention, the inner lumen **282** contains valve **212.** In one embodiment if the present invention, valve **212** is a 3-way valve, infusion, aspiration, and closed, **213.** In another embodiment of the present invention, the valve **212** only opens when pushed by the tip of an access device, e.g., a syringe, when said access device is inserted into the lumen of said port.

Port body **280** and port segments **215, 222** and **230** can be made of any suitable, biocompatible material, such as stainless steel, titanium, alloys, silicone, polymers e.g., polyurethanes, PTFE, lightweight polysulphone or of any combination of the above.

In oner embodiment of the present invention, port **200** can be printed using multi-material 3D and 4D printing technologies.

Walls **222** and **230** of port **200** are shaped and made of a material and processed such as to ensure smooth liquid flow with minimal wall friction and minimal turbulence. The size of the angle **225** between the longitudinal axis of conduit **205** and the longitudinal axis of said port body can be chosen depending on the location of implantation of the port and of the preferred direction of the conduit **205,** for example relative to skin surface. Further, angle **225** can be chosen as a function of the technique used (tunneled or not) and of the desired location of skin access.

In one embodiment of the present invention, port **200** is attached to the skin at the location of implantation using an auto-adhesive layer **260,** for example using a hydrocolloid acrylic adhesive. In one embodiment of the present invention, the auto-adhesive provides antimicrobial protection with integrated chlorhexidine gluconate (CHG). After placement, port **200** can be additionally secured in place with sutures to the skin through the port holes **270** provided in the port body for this purpose. In one embodiment of the present invention, the sutures are absorbable or are removed once the self-adhesive layer **260** is safely secured to the skin.

In one embodiment of the present invention, implantable medical mesh **285** is attached to the body **280** of transcutaneous port **200.** Such a mesh bag provides additional anchoring means adapted for tissue ingrowth, tissue encapsulation or tissue adhesion and ensures greater stability and additional protection of the implanted port. In another embodiment of the present invention, the exterior surface of port body **280** is made of such material and processed in such a way as to ensure the additional anchoring means through tissue integration mentioned above.

In one embodiment of the present invention, a protective cap **250** can be attached to body **280** of the transcutaneous port while the port is not in use. In one embodiment of the present invention, the cap is attached (screwed) and detached (unscrewed) by rotation **265** using the threads **290** in port body **280** and the matching threads **275** of cap **250.** In one embodiment of the present invention, the connection at level **240** between cap **250** and body **280** is sealed using matching seals **245.** The seal **245** can be made of different materials, e.g., rubber or silicone. Seal **245** can also be made of different polymers in a matching pair with the seal **235** inside the wall of the body **280.** According to the present invention a matching male-female profile between the seal **245** and the seal **235** is preferably used that prevents fluid from flowing out of the conduit **282** when the cap is attached to the port body. In one embodiment of the present invention, such profile consists of concentric shapes on the seals **245** and **235** that allow the male portion of the profile situated on seal **245** to enter a groove on seal **235,** while cap **250** is rotated for closure.

In one embodiment of the present invention, protective cap **250** has a safety mechanism to prevent accidental opening. In one embodiment of the present invention the safety mechanism is an anti-rotation lock **257.** To unlock the cap, the lateral push-to-rotate **255** fixtures are used. Other anti-rotation locks can also be used: down-push and rotate, alignment of cap and body to a certain unique position, an external lock that needs to be pushed to release the cap for rotation, or an external lock or key of a shape that only matches a corresponding shape imprinted in the cap.

In one embodiment of the present invention, protective cap **250** is color coded. In one embodiment of the present invention, the surface **251** of the cap **250** can be red to indicated that the port is used to access an artery in the patient's body. In another embodiment, the cap surface **251** of the cap **250** can be blue to indicate that the port is used to access a vein in the patient's body.

In one embodiment of the present invention, protective cap **250** is sterile and single use.

In one embodiment of the present invention, protective cap **250** can be made of any suitable material such as silicone, rubber, or polymers (e.g., polyurethanes, PTFE) or a combination of the above. Stainless steel or alloys like nitinol can be used for the seal **245** or for providing any required reinforcements.

In one embodiment of the present invention, a patch or gel pad **298** is applied over cap and over the surrounding skin to provide additional barrier to microbes and external contaminants and to reduce port colonization and CRSBI (catheter related blood stream infections). In a preferred method of use, patch **298** is to be replaced with each use of the transcutaneous port together with cap **250.**

In one embodiment of the present invention, transcutaneous port **200** provides variable blood flow characteristics in order to accommodate either arterial or venous flow. In one embodiment of the present invention, inner lumen **282** has a smaller diameter at its distal end compared to a larger diameter at its proximal end in order to increase the pressure of fluid infusion into conduit **205** or to decrease the pressure of fluid aspiration from conduit **205.** In another embodiment of the present invention, inner lumen **282** has a larger diameter at its distal end compared to a smaller diameter at its proximal end in order to decrease the pressure of fluid infusion into conduit **205** or to increase the pressure of fluid aspiration from conduit **205.**

In one embodiment of the present invention, transcutaneous port **200** can be detached from the conduit **205** and from the skin **202** and can be replaced with another port **200** or with any other port connectable to conduit **205.**

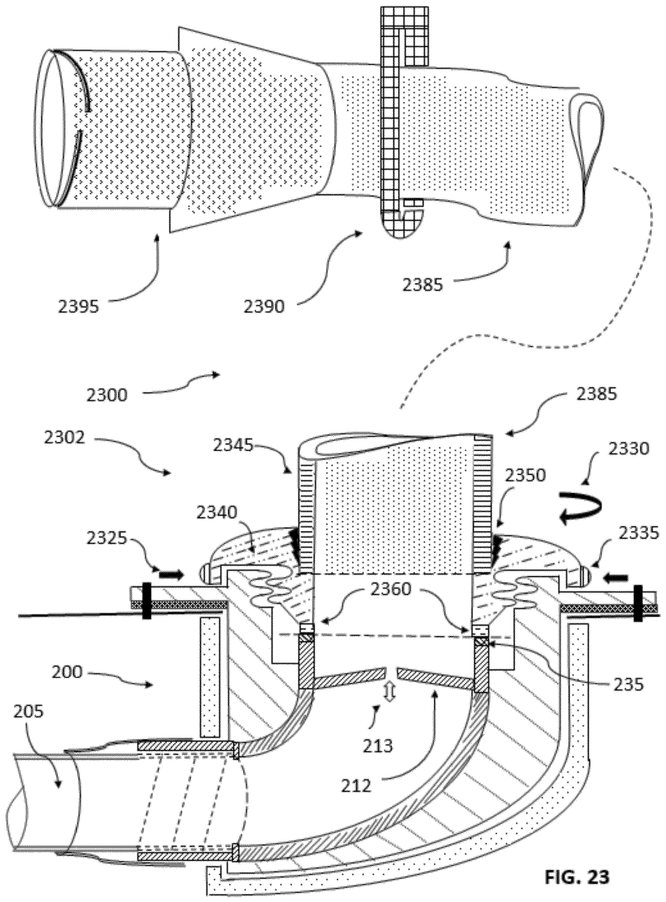

FIG. **23** illustrates an example embodiment of a device and method for needlelessly connecting a patient to a dialyzer according to the present invention wherein an extracorporeal connector **2300** is attached to a transcutaneous port **200.** In one embodiment of the present invention, the extracorporeal connector **2300** consists of a body **2302** that can be attached to transcutaneous port **200,** of a conduit **2385,** and of a standard female Luer connector **2395** with a clamp **2390.** The female Luer connector **2395** can be connected to an external device, e.g., a syringe or a hemodialysis machine. The clamp **2390** can be used to stop the fluid flow through the conduit **2385.** When connected to a syringe, blood can be drawn from, and fluids can be delivered to the blood vessel connected to intracorporeal conduit **205** through port **200.**

In one embodiment of a hemodialysis method according to the present invention, a first intracorporeal conduit **205** connected to a first port **200** is anastomosed to a vein and a second intracorporeal conduit **205** connected to a second port **200** is anastomosed to an artery. A first extracorporeal connector **2300** connected to the first venous access port **200** is connected to the venous line of a dialyzer and a second extracorporeal connector **2300** connected to the second arterial access port **200** is connected to the arterial line of a dialyzer. In one embodiment of the present invention, one or more elements of the connectors **2300** are color coded in blue as to identify venous line access and connection and in red as to identify arterial line access and connection. When the transcutaneous port **200** is in use, i.e., when connected to an extracorporeal connector **2300,** the patch **298** and the cap **250** have been removed and replaced by the body **2302** of the extracorporeal connector **2300.**

The conduit **2385** is connected to the element **2340** of the connector body **2302** through the connecting segment **2350.** Depending on the materials of the conduit wall **2345** and of the body element **2340,** connecting segment **2350** can be made using different technologies and materials, e.g., stainless steel, titanium, alloys, silicone, polymers e.g., polyurethanes, PTFE, lightweight polysulphone or of any combination of the above. In one embodiment of the present invention, conduit **2385** is glued to connector body element **2340.**

In one embodiment of the present invention, element **2340** of the connector body is attached and removed to and from port **200** by rotating **(2330)** and pushing **(2325)** a push-rotate locking mechanism **2335.** The safety mechanism **2335** that prevents accidental disengagement of the element **2340** matches the safety mechanism of port **200.** In one embodiment of the present invention, safety mechanism **2335** is lateral-push-to-rotate anti-rotation lock. Other anti-rotation locks can also be used: down-push and rotate, alignment of cap and body to a certain unique position, or an external lock that needs to be pushed to release the cap for rotation.

The extracorporeal connector **2300** can be made of any suitable material such as silicone or polymers (e.g., polyurethanes, PTFE) or a combination of the above. Stainless steel or alloys like nitinol can be used for braiding the conduit **2385,** for the seals **2360** and **235** or for providing any required reinforcements. Rubber or lightweight polysulphone can be used for the element **2340** of the body **200.**

In one embodiment of the present invention, valve **212** is a 3-way valve **213** (closed, infusion, aspiration) that is normally closed and opens only under the negative pressure (aspiration) or positive pressure (infusion) generated by the device or dialyzer attached to connector **2395.**

In one embodiment of the present invention, valve **212** is normally closed and opens only when the distal end of the extracorporeal connector **2300** is attached to the connector body **2302** such that the distal end of said connector pushes through valve **212** and opens it.

In one embodiment of the present invention, the extracorporeal connector **2300** is sterile and single use.

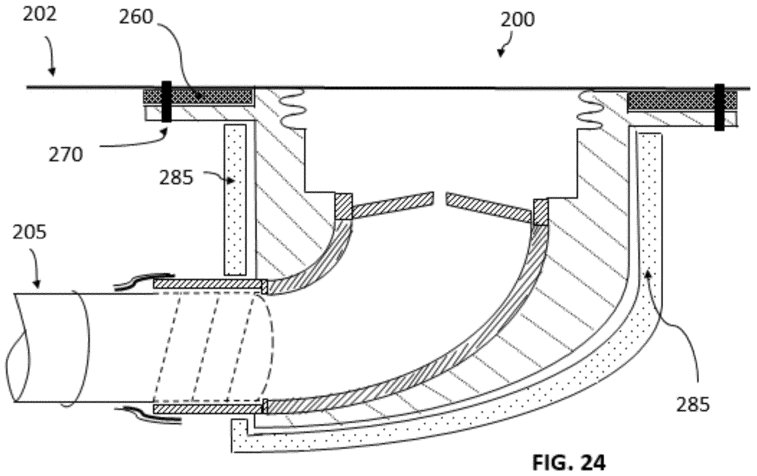

FIG. **24** illustrates another example embodiment of transcutaneous access port **200** placed subcutaneously under the patient's skin **202** and connected to intracorporeal conduit **205** according to the present invention. When said transcutaneous access port **200** is placed subcutaneously, the patient's skin covers said access port and protection patch **298** and protective cap **250** at FIG. **2** are no longer needed. Port **200** can be attached to the patient's skin using sutures **270** or adhesives **260** and/or fixed subcutaneously in the patient's body with an appropriate layer of medical mesh **285.**

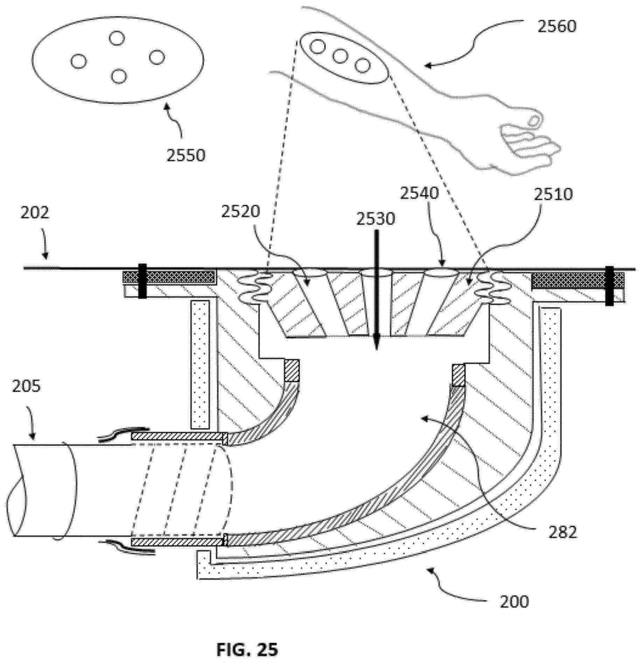

FIG. **25** illustrates another example embodiment of transcutaneous access port **200** placed subcutaneously and comprising an adaptor **2510** having one or several predefined needle access points or channels or buttonholes **2520** to provide one or more fluid paths to intracorporeal conduit **205** through the port's inner lumen **282** according to the present invention. Adaptor **2510** can be attached to (screwed into) port **200** prior to implantation and can be removed by unscrewing it through a skin incision. Each access channel **2520** has a more prominent proximal ending **2540** that can be sensed through patient's skin **202** to identify the point of needle insertion. By inserting a needle through the skin into a channel **2520,** fluid can be infused or aspirated through said channel to and from the inner lumen **282** of port **200** and thus, to and from the intracorporeal conduit **205.** In another embodiment of the present invention, channel **2520** is used to guide **2530** a needle or another type of the access device from the skin **202** into the main lumen **282** of said port **200.**

In one embodiment of the present invention, adaptor **2510** is provided in different configurations as illustrated by **2550** and **2560** with channels or buttonholes **2520** having different locations for each configuration such that, when a different configuration is used, the access points on skin for needle puncture change locations. Thus, the skin is not punctured in the same location each time giving the skin time to heal from one puncture to the next, e.g., from one hemodialysis session to the next. Furthermore, each configuration of adaptor **2510** can have one or several access channels and an appropriate size corresponding to the number and location of the access channels.

In one embodiment of the present invention, adaptor **2510** is made of any suitable, biocompatible material, such as stainless steel, titanium, alloys, silicone, polymers e.g., polyurethanes, PTFE, lightweight polysulphone or of any combination of the above. For example, the body of adaptor **2510** can be made of polymers overmolded over channels **2520** made of stainless steel, titanium, or nitinol.

## Claims

1. A transcutaneous access port for hemodialysis (200) comprising a port body (280) with a subcutaneous segment (215) attachable to an intracorporeal conduit, said transcutaneous access port having an inner lumen profile (282), **characterized in that** the said inner lumen has tapering walls with a larger diameter at one end of said inner lumen and a smaller diameter at the other end of said inner lumen and a smooth transition between the larger and the smaller ends of said inner lumen such as to provide variable blood flow characteristics adapted to either arterial or venous blood flow while minimizing blood flow turbulences.

2. The transcutaneous access port according to claim **1, characterized in that** the said transcutaneous access port comprises a valve (212).

3. The transcutaneous access port according to claim **2, characterized in that** the said valve is configured to open when negative pressure or positive pressure is applied through the inner lumen of said port.

4. The transcutaneous access port according to claim **2, characterized in that** the said valve is configured to open when a device is pushed into said port body through said valve.

5. The transcutaneous access port according to claim **1, characterized in that** the said transcutaneous access port is configured to be implanted subcutaneously and further comprises a removable adaptor (2510), said adaptor including one or more access channels (2520) to the inner lumen of said transcutaneous port that are covered by the skin, can be identified as access points through the skin (2540), and can guide an access needle from the skin into the inner lumen of said port (2530).

6. The transcutaneous access port according to claim **5, characterized in that** the said access channels of said removable adaptor and their corresponding access points under the skin are provided in different configurations at different locations under the skin (2550) in order for needle punctures to be done at different locations on the skin as to allow the skin to heal between one needle puncture to the next.

7. The transcutaneous access port according to claim **1, characterized in that** the said access port comprises an extracorporeal protection cap (250).

8. The transcutaneous access port according to claim **7, characterized in that** the said cap is color-coded to allow for easy identification of blood vessel connections.

9. The transcutaneous access port according to claim **7, characterized in that** the said cap comprises a safety mechanism (255) to prevent accidental opening and a ring-shaped seal (245) for fluid tight connection to the body of said access port.

10. The transcutaneous access port according to claim **1, characterized in that** the inner diameter of the inner lumen (282) is smaller at its distal end compared to a larger diameter at its proximal end in order to increase the pressure of the fluid infusion through the connected intracorporeal conduit and whereby the transition between the smaller and the larger diameters is smooth in order to minimize fluid flow turbulences.

11. The transcutaneous access port according to claim **1, characterized in that** the inner diameter of the inner lumen (282) is larger at its distal end compared to a smaller diameter at its proximal end in order to decrease the pressure of the fluid infusion through the connected intracorporeal conduit and whereby the transition between the smaller and the larger diameters is smooth in order to minimize fluid flow turbulences.

12. The transcutaneous access port according to claim **1, characterized in that** the implantable medical mesh (285) is attached to the outside wall of said device, said mesh providing additional anchoring means adapted for tissue ingrowth, tissue encapsulation or tissue adhesion and ensures greater stability and additional protection of said implanted device.

13. The transcutaneous access port according to claim **1, characterized in that** the said transcutaneous access port further comprises an extracorporeal connector (2300) to provide needleless connection to a dialyzer, said extracorporeal connector comprising a conduit (2385) with a proximal end and a distal end, wherein said distal end includes a matching connector (2302) that can be attached to the device of claim 9 and said proximal end (2395) can be attached to a dialyzer.

14. The transcutaneous access port according to claim **1, characterized in that** the longitudinal axis of the inner lumen of said subcutaneous segment and the longitudinal axis of the inner lumen of said port body can be configured at different angles (225) with respect to each other to adapt to the anatomy of the access and implant locations.

## Patentansprüche

1. Ein transkutaner Zugangsport für die Hämodialyse (200), bestehend aus einem Portkörper (280) mit einem subkutanen Segment (215), das an eine intrakorporale Leitung anschließbar ist, wobei der transkutane Zugangsport ein Innenlumenprofil (282) aufweist, **dadurch gekennzeichnet, dass** das Innenlumen verjüngte Wände mit einem größeren Durchmesser an einem Ende und einem kleineren Durchmesser am anderen Ende aufweist, wobei der Übergang zwischen dem größeren und dem kleineren Ende des Innenlumens ist fließend, um variable Blutflusseigenschaften zu gewährleisten, die an den arteriellen oder venösen Blutfluss angepasst sind und gleichzeitig Blutflussturbulenzen minimieren.

2. Der transkutane Zugangsport nach Anspruch **1, dadurch gekennzeichnet, dass** der transkutane Zugangsport ein Ventil (212) umfasst.

3. Der transkutane Zugangsport nach Anspruch **2, dadurch gekennzeichnet, dass** das Ventil so konfiguriert ist, dass es sich öffnet, wenn durch das Innenlumen des Zugangsports negativen Druck oder positiven Druck angelegt wird.

4. Der transkutane Zugangsport nach Anspruch **2, dadurch gekennzeichnet, dass** sich das Ventil öffnet, wenn eine Vorrichtung durch das Ventil in den Portkörper eingeführt wird.

5. Der transkutane Zugangsport nach Anspruch **1, dadurch gekennzeichnet, dass** der transkutane Zugangsport für die subkutane Implantation konfiguriert ist und einen abnehmbaren Adapter (2510) umfasst, wobei dieser Adapter einen oder mehrere Zugangskanäle (2520) zum inneren Lumen des transkutanen Ports aufweist, die von der Haut bedeckt sind, als Zugangspunkte durch die Haut (2540) identifiziert werden können und eine Zugangsnadel von der Haut in das innere Lumen des Ports (2530) führen können.

6. Der transkutane Zugangsport nach Anspruch **5, dadurch gekennzeichnet, dass** die Zugangskanäle des abnehmbaren Adapters und ihre entsprechenden Zugangspunkte unter der Haut in unterschiedlichen Konfigurationen an verschiedenen Stellen unter der Haut (2550) vorgesehen sind, um Nadelstiche an verschiedenen Stellen der Haut durchführen zu können und so der Haut die Heilung von einem Nadelstich zu dem nächsten zu ermöglichen.

7. Der transkutane Zugangsport nach Anspruch **1, dadurch gekennzeichnet, dass** der transkutane Zugangsport eine extrakorporale Schutzkappe (250) umfasst.

8. Der transkutane Zugangsport nach Anspruch **7, dadurch gekennzeichnet, dass** die Schutzkappe farbcodiert ist, um die Identifizierung von Blutgefäßverbindungen zu erleichtern.

9. Der transkutane Zugangsport nach Anspruch **7, dadurch gekennzeichnet, dass** die Schutzkappe einen Sicherheitsmechanismus (255) zum Verhindern eines unbeabsichtigten Öffnens und eine ringförmige Dichtung (245) für eine flüssigkeitsdichte Verbindung mit dem Körper des Zugangsports umfasst.

10. Der transkutane Zugangsport nach Anspruch **1, dadurch gekennzeichnet, dass** der Innendurchmesser des inneren Lumens (282) an seinem distalen Ende kleiner ist als an seinem proximalen Ende, um den Druck der Flüssigkeitsinfusion durch die angeschlossene intrakorporale Leitung zu erhöhen, und wobei der Übergang zwischen dem kleineren und dem größeren Durchmesser glatt ist, um Turbulenzen im Flüssigkeitsfluss zu minimieren.

11. Der transkutane Zugangsport nach Anspruch **1, dadurch gekennzeichnet, dass** der Innendurchmesser des inneren Lumens (282) an seinem distalen Ende größer ist als an seinem proximalen Ende, um den Druck der Flüssigkeitsinfusion durch die angeschlossene intrakorporale Leitung zu verringern, und wobei der Übergang zwischen dem kleineren und dem größeren Durchmesser glatt ist, um Turbulenzen im Flüssigkeitsfluss zu minimieren.

12. Der transkutane Zugangsport nach Anspruch **1, dadurch gekennzeichnet, dass** das implantierbare medizinische Netz (285) an der Außenwand des Zugangsports befestigt ist, sodass dieses Netz zusätzliche Verankerungselemente für Gewebeeinwuchs, Gewebeeinkapselung oder Gewebeadhäsion bereitstellt und so eine höhere Stabilität und zusätzlichen Schutz des implantierten Zugangsports gewährleistet.

13. Der transkutane Zugangsport nach Anspruch **1, dadurch gekennzeichnet, dass** der transkutane Zugangsport zusätzlich einen extrakorporalen Konnektor (2300) zur nadellosen Verbindung mit einem Dialysator umfasst, wobei dieser extrakorporale Konnektor aus einer Leitung (2385) mit einem proximalen und einem distalen Ende besteht, und das distale Ende einen passenden Konnektor (2302) aufweist, der an den Zugangsport nach Anspruch 9 angeschlossen werden kann, und das proximale Ende (2395) an einen Dialysator angeschlossen werden kann.

14. Der transkutane Zugangsport nach Anspruch **1, dadurch gekennzeichnet, dass** die Längsachse des inneren Lumens des subkutanen Segments und die Längsachse des inneren Lumens des Portkörpers in unterschiedlichen Winkeln (225) zueinander angeordnet werden können, um sich an die Anatomie der Zugangs- und Implantationsstellen anzupassen.

## Revendications

1. Un dispositif d'accès transcutané pour hémodialyse (200) comprenant un corps de dispositif (280) doté d'un segment sous-cutané (215) pouvant être fixé à un conduit intracorporel, ledit dispositif d'accès transcutané ayant une lumière interne (282), **caractérisé en ce que** ladite lumière interne présente des parois coniques de diamètre supérieur à une extrémité et de diamètre inférieur à l'autre extrémité de ladite lumière interne, ainsi qu'une transition douce entre l'extrémité la plus large et l'extrémité la plus petite de ladite lumière interne, de manière à offrir des caractéristiques de débit sanguin variables, adaptées au flux sanguin artériel ou veineux, tout en minimisant les turbulences du flux sanguin.

2. Le dispositif d'accès transcutané selon la revendication **1, caractérisé en ce que** ledit dispositif d'accès transcutané comporte une valve (212).

3. Le dispositif d'accès transcutané selon la revendication **2, caractérisé en ce que** ladite valve est configurée pour s'ouvrir lorsqu'une pression négative ou une pression positive est appliquée à travers la lumière interne de dudit dispositif.

4. Le dispositif d'accès transcutané selon la revendication **2, caractérisé en ce que** ladite valve est configurée pour s'ouvrir lorsqu'un dispositif est poussé dans ledit corps du dispositif par ladite valve.

5. Le dispositif d'accès transcutané selon la revendication **1, caractérisé en ce que** ledit dispositif d'accès transcutané est configuré pour être implanté en sous-cutané et comporte en outre un adaptateur amovible (2510), ledit adaptateur comprenant un ou plusieurs canaux d'accès (2520) à la lumière interne dudit dispositif d'accès transcutané, qui sont recouverts par la peau, pouvant être identifiés comme des points d'accès à travers la peau (2540) et qui peuvent guider une aiguille d'accès de la peau vers la lumière interne dudit dispositif (2530).

6. Le dispositif d'accès transcutané selon la revendication **5, caractérisé en ce que** lesdites canaux d'accès dudit adaptateur amovible et leurs points d'accès correspondants sous la peau sont prévus selon différentes configurations à différents emplacements sous la peau (2550) afin de permettre la réalisation de ponctions avec une aguille d'accès à différents endroits de la peau et de permettre la cicatrisation de la peau entre une ponction et la suivante.

7. Le dispositif d'accès transcutané selon la revendication **1, caractérisé en ce que** ledit dispositif comprend un capuchon de protection extracorporel (250).

8. Le dispositif d'accès transcutané selon la revendication **7, caractérisé en ce que** ledit capuchon est codé par couleur pour faciliter l'identification des connexions des vaisseaux sanguins.

9. Le dispositif d'accès transcutané selon la revendication **7, caractérisé en ce que** ledit capuchon comporte un mécanisme de sécurité (255) pour empêcher toute ouverture accidentelle et un joint annulaire (245) pour une connexion étanche aux fluides avec le corps dudit dispositif d'accès transcutané.

10. Le dispositif d'accès transcutané selon la revendication **1, caractérisé en ce que** le diamètre intérieur de la lumière interne (282) est plus petit à son extrémité distale par rapport à un diamètre plus grand à son extrémité proximale afin d'augmenter la pression d'une perfusion de fluide à travers le conduit intracorporel connecté et de sorte que la transition entre les diamètres plus petit et plus grand est douce afin de minimiser les turbulences d'écoulement de fluide.

11. Le dispositif d'accès transcutané selon la revendication **1, caractérisé en ce que** le diamètre intérieur de la lumière interne (282) est plus grand à son extrémité distale par rapport à un diamètre plus petit à son extrémité proximale afin de diminuer la pression d'une perfusion du fluide à travers le conduit intracorporel connecté et de sorte que la transition entre le diamètre le plus petit et le diamètre le plus grand est douce afin de minimiser les turbulences d'écoulement du fluide.

12. Le dispositif d'accès transcutané selon la revendication **1, caractérisé en ce qu'**un treillis médical implantable (285) est fixé à la paroi extérieure dudit dispositif, ledit treillis médical implantable fournissant des moyens d'ancrage supplémentaires adaptés à la croissance tissulaire, à l'encapsulation tissulaire ou à l'adhésion tissulaire et assurant une plus grande stabilité et une protection supplémentaire dudit dispositif implanté.

13. Le dispositif d'accès transcutané selon la revendication **1, caractérisé en ce que** ledit dispositif d'accès transcutané comporte en outre un connecteur extracorporel (2300) permettant une connexion sans aiguille à un dialyseur ledit connecteur extracorporel comprenant un conduit (2385) doté d'une extrémité proximale et d'une extrémité distale, dans lequel ladite extrémité distale inclut un connecteur correspondant (2302) qui peut être fixé au dispositif selon la revendication 9 et ladite extrémité proximale (2395) peut être fixée à un dialyseur.

14. Le dispositif d'accès transcutané selon la revendication **1, caractérisé en ce que** l'axe longitudinal de la lumière interne dudit segment sous-cutané et l'axe longitudinal de la lumière interne du corps dudit dispositif peuvent être configurés à des angles différents (225) l'un par rapport à l'autre afin de s'adapter à l'anatomie des emplacements d'accès et d'implantation.
